(19) Europäisches Patentamt
European Patent Office
Office européen des brevets

(11) Veröffentlichungsnummer: **0 648 750 A2**

# (12) EUROPÄISCHE PATENTANMELDUNG

(21) Anmeldenummer: **94115561.6**

(22) Anmeldetag: **04.10.94**

(51) Int. Cl.[6]: **C07D 241/24**, C07D 409/12, A01N 43/60, //C07D237/24, C07D239/28,A01N43/58, A01N43/54

(30) Priorität: **12.10.93 DE 4334706**

(43) Veröffentlichungstag der Anmeldung: **19.04.95 Patentblatt 95/16**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**D-67063 Ludwigshafen (DE)**

(72) Erfinder: **Rentzea, Costin, Dr.**
**Richard-Kuhn-Strasse 1-3**
**D-69123 Heidelberg (DE)**
Erfinder: **Harreus, Albrecht, Dr.**
**Teichgasse 13**
**D-67063 Ludwigshafen (DE)**
Erfinder: **Kirstgen, Reinhard, Dr.**
**Erkenbrechtstrasse 23e**
**D-67434 Neustadt (DE)**
Erfinder: **Ammermann, Eberhard, Dr.**
**Von-Gagern-Strasse 2**
**D-64646 Heppenheim (DE)**
Erfinder: **Lorenz, Gisela, Dr.**
**Erlenweg 13**
**D-67434 Neustadt (DE)**

(54) **Pyridin- und Diazincarbonsäureamidderivate, Verfahren zu ihrer Herstellung, und ihre Verwendung als Fungizide.**

(57) Pyridin- und Diazincarbonsäurederivate der Formeln Ia und Ib,

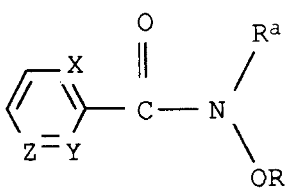

Ia

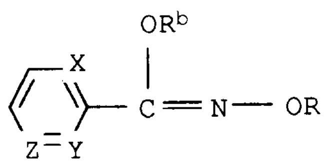

Ib

in der die Substituenten die folgende Bedeutung haben:

X, Y und Z — CH oder N, wobei eine oder zwei der Variablen CH bedeutet und eine der Variablen zusätzlich $N^+-O^-$ bedeuten kann und wobei Z nicht N oder $N^+-O^-$ bedeutet, wenn X und Y gleichzeitig für CH stehen;

R — $C_3$-$C_8$-Alkyl, $C_2$-$C_8$-Halogenalkyl, $C_3$-$C_8$-Alkenyl, $C_3$-$C_8$-Halogenalkenyl oder $C_3$-$C_8$-Alkinyl; $C_1$-$C_{10}$-Alkyl oder $C_3$-$C_{10}$-Alkenyl, welches einen Aryl-, Aryloxy-, Arylthio-, Heteroaryl-, Heteroaryloxy-oder Heteroarylthiorest trägt, wobei der Aryl- bzw. der Heteroarylrest seinerseits ein bis fünf Halogenatome und/oder ein bis drei der folgenden Gruppen tragen kann: Nitro, Cyano, $C_1$-$C_4$-Alkyl, $C_1$-$C_4$-Halogenalkyl, $C_3$-$C_4$-Alkenyl, $C_1$-$C_4$-Alkoxy, $C_1$-$C_4$-Halogenalkoxy, $C_3$-$C_4$-Alkenyloxy oder $C_1$-$C_4$-Alkylthio,

EP 0 648 750 A2

$R^a$ Wasserstoff, $C_1$-$C_4$-Alkyl, $C_3$-$C_4$-Alkenyl oder $C_3$-$C_4$-Alkinyl;
$R^b$ $C_1$-$C_4$-Alkyl, $C_3$-$C_4$-Alkenyl oder $C_3$-$C_4$-Alkinyl,

sowie deren Salze, Verfahren zu iherer Herstellung, sie enthaltende Mittel und ihre Verwendung als Fungizide.

Die vorliegende Erfindung betrifft Pyridin- und Diazincarbonsäurederivate der Formeln Ia und Ib,

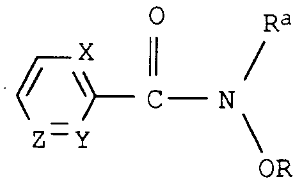

Ia

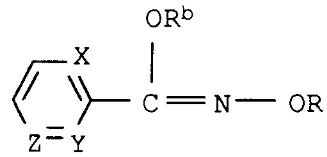

Ib

in der die Substituenten die folgende Bedeutung haben:

- X, Y und Z: CH oder N, wobei eine oder zwei der Variablen CH bedeutet und eine der Variablen zusätzlich $N^{+}$-$O^{-}$ bedeuten kann und wobei Z nicht N oder $N^{+}$-$O^{-}$ bedeutet, wenn X und Y gleichzeitig für CH stehen;
- R: $C_3$-$C_8$-Alkyl, $C_2$-$C_8$-Halogenalkyl, $C_3$-$C_8$-Alkenyl, $C_3$-$C_8$-Halogenalkenyl oder $C_3$-$C_8$-Alkinyl; $C_1$-$C_{10}$-Alkyl oder $C_3$-$C_{10}$-Alkenyl, welches einen Aryl-, Aryloxy-, Arylthio-, Heteroaryl-, Heteroaryloxy- oder Heteroarylthiorest trägt, wobei der Aryl- bzw. der Heteroarylrest seinerseits ein bis fünf Halogenatome und/oder ein bis drei der folgenden Gruppen tragen kann: Nitro, Cyano, $C_1$-$C_4$-Alkyl, $C_1$-$C_4$-Halogenalkyl, $C_3$-$C_4$-Alkenyl, $C_1$-$C_4$-Alkoxy, $C_1$-$C_4$-Halogenalkoxy, $C_3$-$C_4$-Alkenyloxy oder $C_1$-$C_4$-Alkylthio,
- $R^a$: Wasserstoff, $C_1$-$C_4$-Alkyl, $C_3$-$C_4$-Alkenyl oder $C_3$-$C_4$-Alkinyl;
- $R^b$: $C_1$-$C_4$-Alkyl, $C_3$-$C_4$-Alkenyl oder $C_3$-$C_4$-Alkinyl,

sowie deren Salze.

Außerdem wurden Verfahren zu ihrer Herstellung, sie enthaltende Mittel und ihre Verwendung zur Bekämpfung von Schadpilzen gefunden.

Aufgabe der vorliegenden Erfindung waren neue Verbindungen mit verbessertem und verbreitertem Wirkungsspektrum.

Demgemäß wurden die eingangs definierten Pyridin- und Diazincarbonsäurederivate der Formeln Ia und Ib gefunden. Außerdem wurden Verfahren zur Herstellung dieser Verbindungen, sie enthaltende Mittel und ihre Verwendung zur Bekämpfung von Schadpilzen gefunden.

Die erfindungsgemäßen Verbindungen sind auf verschiedenen, in analoger Weise aus der Literatur bekannten, Wegen zugänglich.

So erhält man die Verbindungen der Formel Ia beispielsweise dadurch, daß man ein Hydroxylamin der Formel II in an sich bekannter Weise mit einem Carbonsäurederivat der Formel III umsetzt.

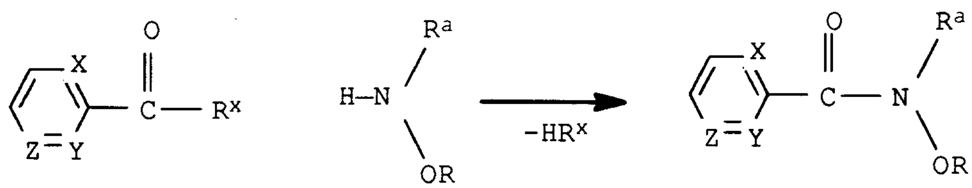


III II Ia

Der Rest $R^x$ in der Formel III steht für ein Halogenatom wie Fluor, Chlor, Brom und Jod, oder eine $C_1$-$C_2$-Alkoxygruppe (Methoxy und Ethoxy), vorzugsweise Chlor, Brom und Methoxy.

Die Umsetzung erfolgt üblicherweise bei Temperaturen von -20 °C bis 150 °C, vorzugsweise 0 °C bis 150 °C, insbesondere 20 °C bis 130 °C in einem inerten organischen Lösungsmittel.

Als Lösungsmittel eignen sich beispielsweise Halogenkohlenwasserstoffe, insbesondere Chlorkohlenwasserstoffe, z. B. 1,1,2,2-Tetrachlorethylen, oder 1,1,2,2-Tetrachlorethan, Dichlorpropan, Methylenchlorid, Dichlorbutan, 1,1,1- oder 1,1,2-Trichlorethan, Trichlorethylen, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol; Ether, z. B. Ethylpropylether, Methyl-tert.butylether, n-Butylethylether, Di-n-Butylether, Di-isobutylether, Diisoamylether, Diisopropylether, Anisol, Phenethol, Cyclohexylmethylether, Diethylether, Ethylenglykoldimethylether, Tetrahydrofuran, Dioxan, Thioanisol; Alkohole

wie Methanol, Ethanol, Propanol, iso-Propanol, n-Butanol, isoButanol, tert.-Butanol, Ethylenglykol und Propandiole; Nitrile wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z. B. Ethylacetat, Acetessigester, Isobutylacetat; Amide, z. B. Formamid, Methylformamid, Dimethylformamid, gegebenenfalls auch Wasser und entsprechende Gemische. Als Lösungsmittel können auch die Verbindungen der Formel II im Überschuß verwendet werden. Zweckmäßig verwendet man das Lösungsmittel in einer Menge von 100 bis 2000 Gew.-%, vorzugsweise von 200 - 700 Gew.-%, bezogen auf Ausgangsstoff III.

Als Basen kommen beispielsweise Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-4-aminopyridin, N,N-Diethyl-4-aminopyridin, N,N-Dipropyl-4-aminopyridin, N,N-Dipropyl-4-aminopyridin, N-Methylpyrrolidin, N-Ethylpyrrolidin, N-Methylpiperidin, N-Ethylpiperidin, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, beta-Picolin, Isochinolin, N,N,N',N'-Tetramethylethylendiamin, N,N,N',N'-Tetraethylethylendiamin, N-Propyldiisopropylamin, N,N-Dimethylcyclohexylamin, 2,6-Lutidin, 2,4-Lutidin, Triethylendiamin in Betracht.

Zweckmäßig verwendet man die Base in stöchiometrischen Mengen, im Überschuß oder Unterschuß von jeweils bis zu 20 Mol.-%, bezogen auf den Ausgangsstoff III.

Außerdem kann es von Nutzen sein, die Umsetzung in Gegenwart eines Katalysators durchzuführen.

In diesem Sinne kommen vorzugsweise 4-Dimethylaminopyridin und 4-Pyrrolidinopyridin in Betracht (J. Cossy et al., Synthesis, 753f (1989)).

Ausgangsstoffe der Formel II sind aus EP-A 368 255 und EP-A 456 112 bekannt (siehe auch: Houben-Weyl, Bd. 16a, S. 214ff (1990)).

Im Hinblick auf die Beschleunigung und Vervollständigung der Reaktion kann es von Vorteil sein, die Umsetzung in Gegenwart einer Base und/oder eines Katalysators durchzuführen.

Die Verbindungen der Formel Ib sind in an sich bekannter Weise aus den Verbindungen Ia erhältlich. Man geht dabei im allgemeinen so vor, daß man eine Verbindung der Formel Ia in an sich bekannter Weise mit einer Verbindung der Formel IV umsetzt.

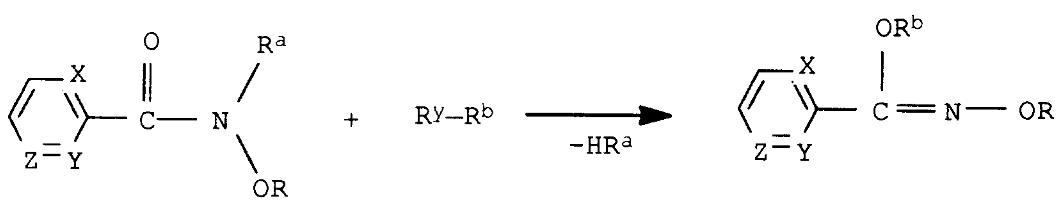


Ia IV Ib

Der Rest $R^y$ in der Formel IV steht für eine nukleophil verdrängbare Abgangsgruppe, besonders Halogen wie insbesondere Chlor und Brom sowie Alkenyl-, Alkoxy- oder Arylsulfonylreste.

Die Umsetzung erfolgt im allgemeinen bei Temperaturen von -20 °C bis 130 °C, vorzugsweise 10 °C bis 100 °C, insbesondere 10 °C bis 90 °C in einem inerten Lösungsmittel in Gegenwart einer Base.

Als Lösungsmittel eignen sich beispielsweise die genannten; insbesondere kommen Essigsäureethylester, Methylenchlorid, Toluol, Tetrahydrofuran, Dioxan, Aceton, Methylethylketon, Methyl-tert.butylether, Diethylether, Dimethylformamid, Dimethylacetamid oder deren Gemische in Betracht.

Als Basen kommen in diesem Verfahren neben den vorstehend genannten Natriumhydrid, Kalium-tert.-butylat und Natriummethylat in Betracht.

Im Hinblick auf ihre biologische Wirkung gegen Schadpilze kommen Verbindungen der Formeln Ia und Ib in Betracht, in denen die Substituenten die folgende Bedeutung haben:

X CH oder N,

Y CH oder N,

Z CH, N oder $N^+$-$O^-$, wobei Z nicht N oder $N^+$-$O^-$ bedeutet, wenn X und Y gleichzeitig für CH stehen,

wobei X, Y und Z nicht gleichzeitig für CH und nicht gleichzeitig für N stehen.

Besonders bevorzugt sind Verbindungen der Formeln Ia und Ib, in denen der X, Y und Z enthaltende Ring für Pyridin-2-yl, Pyridazin-3-yl, Pyrazinyl, Pyrimidin-2-yl, 1,4-Diazin-2-yl, Pyridin-2-yl-1-N-oxid, Pyridazin-3-yl-1-N-oxid, Pyrazin-3-yl-1-N-oxid oder 1,4-Diazin-2-yl-4-N-oxid, insbesondere 1,4-Diazin-2-yl oder 1,4-Diazin-2-yl-4-N-oxid, steht.

R steht insbesondere für $C_3$-$C_8$-Alkyl, $C_2$-$C_8$-Halogenalkyl, $C_3$-$C_8$-Alkenyl, $C_3$-$C_8$-Halogenalkenyl, $C_3$-$C_8$-Alkinyl oder $C_3$-$C_8$-Halogenalkinyl, oder für $C_1$-$C_{10}$-Alkyl oder $C_3$-$C_{10}$-Alkenyl, welches einen Phenyl-, Phenoxy-, Thienyl-, Thienyloxy-, Furanyl-, Furanyloxy-, Isoxazolyl-, Pyridyl- oder Pyridyloxyrest trägt, wobei dieser Rest seinerseits ein bis fünf Halogenatome und/oder ein bis drei der folgenden Gruppen tragen kann: Nitro, Cyano, $C_1$-$C_4$-Alkyl, $C_1$-$C_4$-Halogenalkyl, $C_3$-$C_4$-Alkenyl, $C_1$-$C_4$-Alkoxy, $C_1$-$C_4$-Halogenalkoxy, $C_3$-$C_4$-Alkenyloxy oder $C_1$-$C_4$-Alkylthio.

In den Verbindungen Ia steht $R^a$ insbesondere für Wasserstoff, Methyl, Ethyl, Propyl, Butyl und 2-Propenyl.

$R^b$ in den Verbindungen Ib bedeutet insbesondere Methyl, Ethyl, Propyl, Butyl und 2-Propenyl.

Als Salze der Verbindungen Ia und Ib kommen beispielsweise Hydrochloride, Nitrate oder Sulfate in Betracht.

Bei den vorstehenden Definitionen der Substituenten wurden unter anderem Sammelbegriffe verwendet, die allgemein repräsentativ für die folgenden Gruppen stehen:

Halogen: Fluor, Chlor, Brom und Jod;

Alkyl: geradkettige oder verzweigte Alkylgruppen mit (soweit nicht anders beschränkt) 1 bis 10, vorzugsweise 1 bis 8, insbesondere 1 bis 6 Kohlenstoffatomen, z.B. Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

Halogenalkyl: geradkettige oder verzweigte Alkylgruppen mit (soweit nicht anders beschränkt) 1 bis 8 Kohlenstoffatomen, wobei in diesen Gruppen teilweise oder vollständig die Wasserstoffatome durch Halogenatome wie vorstehend genannt ersetzt sein können, z.B. $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluor ethyl, 2,2-Dichlor-2-fluorethyl, 2,2,2-Trichlorethyl und Pentafluorethyl;

Alkoxy: geradkettige oder verzweigte Alkylgruppen wie vorstehend genannte, mit 1 bis 4 Kohlenstoffatomen, welche über ein Sauerstoffatom (-O-) an das Gerüst gebunden sind;

Halogenalkoxy: geradkettige oder verzweigte Halogenalkylgruppen wie vorstehend genannt, mit 1 bis 4 Kohlenstoffatomen, welche über ein Sauerstoffatom (-O-) an das Gerüst gebunden sind;

Alkylthio: geradkettige oder verzweigte Alkylgruppen wie vorstehend genannte, mit 1 bis 4 Kohlenstoffatomen, welche über ein Schwefelatom (-S-) an das Gerüst gebunden sind;

Alkenyl: geradkettige oder verzweigte Kohlenwasserstoffgruppen mit (soweit nicht anders beschränkt) 2 bis 10 Kohlenstoffatomen und ein oder zwei Doppelbindungen in beliebigen nicht-kummulierten Positionen, z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2pentenyl, 2-Methyl-2-pentenyl, 3-Methyl-2-pentenyl, 4-Methyl-2- pentenyl, 1-Methyl-3-pentenyl, 2-Methyl-3-pentenyl, 3-Methyl-3- pentenyl, 4-Methyl-3-pentenyl, 1-Methyl-4-pentenyl, 2-Methyl-4- pentenyl, 3-Methyl-4-pentenyl, 4-Methyl-4-pentenyl, 1,1-Dimethyl- 2-butenyl, 1,1-Di-methyl-3-butenyl, 1,2-Dimethyl-1-butenyl, 1,2- Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Eth yl-1-butenyl, 1-Ethyl-2-butenyl, 1-Ethyl-3-butenyl, 2- Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl- 1-prop enyl und 1-Ethyl-2-methyl-2-propenyl;

Halogenalkenyl: geradkettige oder verzweigte Alkenylgruppen wie vorstehend genannt, mit (soweit nicht anders beschränkt) 3 bis 8 Kohlenstoffatomen, wobei in diesen Gruppen teilweise oder vollständig die

Wasserstoffatome durch Halogenatome wie vorstehend genannt ersetzt sein können;
Alkenyloxy: geradkettige oder verzweigte Kohlenwasserstoffgruppen wie vorstehend genannt, mit (soweit nicht anders beschränkt) 3 oder 4 Kohlenstoffatomen und einer Doppelbindung in einer beliebigen Position, welches über eine Sauerstoffgruppe (-O-) an das Gerüst gebunden ist;
Alkinyl: geradkettige oder verzweigte Kohlenwasserstoffgruppen mit (soweit nicht anders beschränkt) 3 bis 8 Kohlenstoffatomen und einer oder zwei Dreifachbindungen in beliebigen nicht-kummulierten Positionen, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1- Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Aryl: eine aromatische Kohlenwasserstoffgruppe wie Phenyl oder Naphthyl, insbesondere Phenyl;
Aryloxy bzw. Arylthio: eine aromatische Kohlenwasserstoffgruppe wie vorstehend genannte, welche über ein Sauerstoff- (-O-) oder ein Schwefelatom (-S-) an das Gerüst gebunden ist;
Hetaryl: ein aromatischer 5- oder 6-gliedriger Heterocyclus, welcher neben Kohlenstoffringgliedern im Falle des 6-Rings ein bis vier Stickstoffatome enthalten kann und welcher im Falle des 5-Rings ein bis drei Stickstoffatome oder eines oder zwei Stickstoffatome und ein Sauerstoff- oder ein Schwefelatom oder ein Sauerstoff- oder ein Schwefelatom als Heteroatom enthalten kann, z.B. Pyridyl, Pyridazinyl, Pyrimidyl, Pyrazinyl, Triazinyl, Furyl, Thienyl, Pyrrolyl, Pyrazolyl, Imidazolyl, Triazolyl, Isoxyzolyl, Oxazolyl, Oxadiazolyl, Isothiazolyl, Thiazolyl und Thiadiazolyl;
Hetaryloxy bzw. Hetarylthio: ein aromatischer 5- oder 6-gliedriger Heterocyclus wie vorstehend genannte, welcher über ein Sauerstoff- (-O-) oder ein Schwefelatom (-S-) an das Gerüst gebunden ist.

Im Hinblick auf ihre biologische Wirkung sind insbesondere die in den Tabellen A und B zusammengestellten Verbindungen Ia und Ib bevorzugt:

Tabelle A

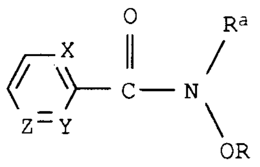

Ia

| X | Y | Z | $R^a$ | R |
|---|---|---|---|---|
| N | CH | N | H | $n-C_3H_7$ |
| N | CH | $N^+-O^-$ | H | $n-C_3H_7$ |
| N | CH | N | $CH_3$ | $n-C_3H_7$ |
| N | CH | $N^+-O^-$ | $CH_3$ | $n-C_3H_7$ |
| N | CH | N | $C_2H_5$ | $n-C_3H_7$ |
| N | CH | N | $n-C_3H_7$ | $n-C_3H_7$ |
| N | CH | N | $n-C_4H_9$ | $n-C_3H_7$ |
| N | CH | $N^+-O^-$ | $n-C_4H_9$ | $n-C_3H_7$ |
| N | CH | N | $-CH_2CH=CH_2$ | $n-C_3H_7$ |
| CH | N | N | H | $n-C_3H_7$ |
| CH | N | $N^+-O^-$ | H | $n-C_3H_7$ |
| CH | N | N | $CH_3$ | $n-C_3H_7$ |
| CH | N | N | $C_2H_5$ | $n-C_3H_7$ |
| CH | N | N | $n-C_3H_7$ | $n-C_3H_7$ |
| CH | N | N | $n-C_4H_9$ | $n-C_3H_7$ |
| CH | N | N | $-CH_2CH=CH_2$ | $n-C_3H_7$ |
| N | CH | N | H | $n-C_4H_9$ |
| N | CH | $N^+-O^-$ | H | $n-C_4H_9$ |
| N | CH | N | $CH_3$ | $n-C_4H_9$ |
| N | CH | N | $C_2H_5$ | $n-C_4H_9$ |
| N | CH | N | $n-C_3H_7$ | $n-C_4H_9$ |
| N | CH | N | H | $iso-C_4H_9$ |
| N | CH | N | $CH_3$ | $iso-C_4H_9$ |
| N | CH | N | $C_2H_5$ | $iso-C_4H_9$ |
| N | CH | N | $n-C_3H_7$ | $iso-C_4H_9$ |
| N | CH | N | H | $sek.-C_4H_9$ |
| N | CH | N | $CH_3$ | $sek.-C_4H_9$ |
| CH | N | N | H | $sek.-C_4H_9$ |
| CH | N | N | $CH_3$ | $sek.-C_4H_9$ |
| N | CH | N | H | $n-C_5H_{11}$ |

| X | Y | Z | $R^a$ | R |
|---|---|---|---|---|
| N | CH | N | $CH_3$ | n-$C_5H_{11}$ |
| N | CH | N | H | iso-$C_5H_{11}$ |
| N | CH | N | $CH_3$ | iso-$C_5H_{11}$ |
| N | CH | N | H | n-$C_6H_{13}$ |
| N | CH | N | $CH_3$ | n-$C_6H_{13}$ |
| N | CH | N | H | n-$C_7H_{15}$ |
| N | CH | N | $CH_3$ | n-$C_7H_{15}$ |
| CH | N | N | H | n-$C_7H_{15}$ |
| CH | N | N | $CH_3$ | n-$C_7H_{15}$ |
| CH | N | N | $C_2H_5$ | n-$C_7H_{15}$ |
| N | CH | N | H | n-$C_8H_{17}$ |
| N | CH | N | $CH_3$ | n-$C_8H_{17}$ |
| N | CH | $N^+-O^-$ | $CH_3$ | n-$C_8H_{17}$ |
| N | CH | N | H | $-CH_2-CH_2-Cl$ |
| N | CH | N | $CH_3$ | $-CH_2-CH_2-Cl$ |
| N | CH | N | $-CH_2CH=CH_2$ | $-CH_2-CH_2-Cl$ |
| N | CH | N | H | $-(CH_2)_3Cl$ |
| N | CH | N | $CH_3$ | $-(CH_2)_3Cl$ |
| N | CH | N | H | $-(CH_2)_4Cl$ |
| N | CH | N | $CH_3$ | $-(CH_2)_4Cl$ |
| N | CH | N | H | $-(CH_2)_5Cl$ |
| N | CH | N | $CH_3$ | $-(CH_2)_5Cl$ |
| N | CH | N | H | $-(CH_2)_5Br$ |
| N | CH | N | $CH_3$ | $-(CH_2)_5Br$ |
| N | CH | N | H | $-(CH_2)_8Cl$ |
| N | CH | N | $CH_3$ | $-(CH_2)_8Cl$ |
| CH | N | N | H | $-(CH_2)_8Cl$ |
| CH | N | N | $CH_3$ | $-(CH_2)_8Cl$ |
| N | CH | N | H | $-CH_2-CF_3$ |
| N | CH | N | $CH_3$ | $-CH_2-CF_3$ |
| N | N | CH | H | $-CH_2-CH_2OCH_3$ |
| N | N | CH | $CH_3$ | $-CH_2-CH_2OCH_3$ |
| CH | N | N | H | $-(CH_2)_3OCH_3$ |
| CH | N | N | $CH_3$ | $-(CH_2)_3OCH_3$ |
| N | CH | N | H | $-(CH_2)_6OCH_3$ |
| N | CH | N | $CH_3$ | $-(CH_2)_6OCH_3$ |
| N | CH | N | H | $-(CH_2)_4OC_2H_5$ |
| N | CH | N | H | $-(CH_2)_5OC_2H_5$ |
| N | CH | N | H | $-(CH_2)_6OC_2H_5$ |

| X | Y | Z | $R^a$ | R |
|---|---|---|---|---|
| CH | CH | N | H | $-CH_2-CH=CH_2$ |
| CH | CH | N | $CH_3$ | $-CH_2-CH=CH_2$ |
| N | CH | N | H | $-CH_2-CH=CH_2$ |
| N | CH | N | $CH_3$ | $-CH_2-CH=CH_2$ |
| N | CH | N | $C_2H_5$ | $-CH_2-CH=CH_2$ |
| CH | N | N | H | $-CH_2-CH=CH-CH_3$ |
| CH | N | N | $CH_3$ | $-CH_2-CH=CH-CH_3$ |
| N | CH | N | H | $-CH_2-C(CH_3)=CH_2$ |
| N | CH | N | $CH_3$ | $-CH_2-C(CH_3)=CH_2$ |
| N | CH | N | H | $-CH_2-CH=C(CH_3)_2$ |
| N | CH | N | $CH_3$ | $-CH_2-CH=C(CH_3)_2$ |
| N | N | CH | H | $-CH_2-CH=CH-C_2H_5$ |
| N | CH | N | H | $-CH_2-CH=CH-C_2H_5$ |
| N | CH | N | H | $-CH_2-CH=CH-C_3H_7$ |
| N | CH | N | $CH_3$ | $-CH_2-CH=CH-C_3H_7$ |
| N | CH | N | H | $-CH_2-CH=CH-CH=CH-C_3H_7$ |
| N | CH | N | $CH_3$ | $-CH_2-CH=CH-CH=CH-C_3H_7$ |
| N | CH | N | H | $-CH_2-C(Br)=CH_2$ |
| N | CH | N | $CH_3$ | $-CH_2-C(Br)=CH_2$ |
| CH | N | N | H | $-CH_2-CH=CH-Cl$ |
| CH | N | N | H | $-CH_2-C(Cl)=CCl_2$ |
| N | CH | N | H | $-CH_2-C(Cl)=CCl_2$ |
| N | CH | N | $CH_3$ | $-CH_2-C(Cl)=CCl_2$ |
| N | CH | N | H | $-CH_2-CH=C(Cl)-CH_3$ |
| N | CH | N | $CH_3$ | $-CH_2-CH=C(Cl)-CH_3$ |
| CH | N | N | $CH_3$ | $-CH_2-C_6H_5$ |
| N | CH | N | H | $-CH_2-C_6H_5$ |
| N | CH | N | $CH_3$ | $-CH_2-C_6H_5$ |
| N | CH | N | $C_2H_5$ | $-CH_2-C_6H_5$ |
| N | CH | N | $C_3H_7$-n | $-CH_2-C_6H_5$ |
| N | CH | N | $-CH_2CH=CH_2$ | $-CH_2-C_6H_5$ |
| N | CH | N | H | $-CH_2-C_6H_4-2F$ |
| N | CH | N | $CH_3$ | $-CH_2-C_6H_4-2F$ |
| N | CH | N | $C_2H_5$ | $-CH_2-C_6H_4-2F$ |
| CH | CH | N | H | $-CH_2-C_6H_4-3F$ |
| N | CH | N | H | $-CH_2-C_6H_4-3F$ |
| N | CH | N | $CH_3$ | $-CH_2-C_6H_4-3F$ |
| N | CH | N | H | $-CH_2-C_6H_4-4F$ |
| N | CH | N | $CH_3$ | $-CH_2-C_6H_4-4F$ |

| X | Y | Z | $R^a$ | R |
|---|---|---|---|---|
| N | CH | N | $C_4H_9$-n | $-CH_2-C_6H_4$-4F |
| N | CH | N | H | $-CH_2-C_6H_3$-2,4-$F_2$ |
| N | CH | N | $CH_3$ | $-CH_2-C_6H_3$-2,4-$F_2$ |
| CH | N | N | H | $-CH_2-C_6H_4$-2Cl |
| CH | N | N | $CH_3$ | $-CH_2-C_6H_4$-2Cl |
| CH | N | N | $C_2H_5$ | $-CH_2-C_6H_4$-2Cl |
| N | CH | N | H | $-CH_2-C_6H_4$-2Cl |
| N | CH | N | $CH_3$ | $-CH_2-C_6H_4$-2Cl |
| N | CH | N | $C_2H_5$ | $-CH_2-C_6H_4$-2Cl |
| N | CH | N | $C_3H_7$-n | $-CH_2-C_6H_4$-2Cl |
| N | CH | N | $C_4H_9$-n | $-CH_2-C_6H_4$-2Cl |
| N | CH | N | $-CH_2CH=CH_2$ | $-CH_2-C_6H_4$-2Cl |
| N | CH | N | H | $-CH_2-C_6H_4$-3Cl |
| N | CH | N | $CH_3$ | $-CH_2-C_6H_4$-3Cl |
| N | CH | N | H | $-CH_2-C_6H_4$-3Cl |
| N | CH | N | $CH_3$ | $-CH_2-C_6H_4$-3Cl |
| N | CH | N | $C_2H_5$ | $-CH_2-C_6H_4$-3Cl |
| N | CH | N | $C_3H_7$-n | $-CH_2-C_6H_4$-3Cl |
| N | CH | N | $-CH_2CH=CH_2$ | $-CH_2-C_6H_4$-3Cl |
| N | CH | N | H | $-CH_2-C_6H_3$-2,4-$Cl_2$ |
| N | CH | N | $CH_3$ | $-CH_2-C_6H_3$-2,4-$Cl_2$ |
| N | CH | N | H | $-CH_2-C_6H_3$-3,4-$Cl_2$ |
| N | CH | N | $CH_3$ | $-CH_2-C_6H_3$-3,4-$Cl_2$ |
| N | CH | N | $C_2H_5$ | $-CH_2-C_6H_3$-3,4-$Cl_2$ |
| N | CH | N | H | $-CH_2-C_6H_3$-2,6-$Cl_2$ |
| N | CH | N | $CH_3$ | $-CH_2-C_6H_3$-2,6-$Cl_2$ |
| N | CH | N | H | $-CH_2-C_6H_4$-4Br |
| N | CH | N | $CH_3$ | $-CH_2-C_6H_4$-4Br |
| N | CH | N | H | $-CH_2-C_6H_4$-2$CH_3$ |
| N | CH | N | $CH_3$ | $-CH_2-C_6H_4$-2$CH_3$ |
| N | CH | N | H | $-CH_2-C_6H_4$-3$CH_3$ |
| N | CH | N | $CH_3$ | $-CH_2-C_6H_4$-3$CH_3$ |
| N | CH | N | H | $-CH_2-C_6H_4$-4$CH_3$ |
| N | CH | N | $CH_3$ | $-CH_2-C_6H_4$-4$CH_3$ |
| N | CH | N | H | $-CH_2-C_6H_4$-4$C_4H_9$-tert. |
| N | CH | N | $CH_3$ | $-CH_2-C_6H_4$-4$C_4H_9$-tert. |
| N | CH | N | H | $-CH_2-C_6H_4$-2$OCH_3$ |
| N | CH | N | $CH_3$ | $-CH_2-C_6H_4$-2$OCH_3$ |
| N | CH | N | H | $-CH_2-C_6H_4$-3$OCH_3$ |

| X | Y | Z | $R^a$ | R |
|---|---|---|---|---|
| N | CH | N | $CH_3$ | $-CH_2-C_6H_4-3OCH_3$ |
| N | CH | N | H | $-CH_2-C_6H_4-4OCH_3$ |
| N | CH | N | $CH_3$ | $-CH_2-C_6H_4-4OCH_3$ |
| N | CH | N | $C_2H_5$ | $-CH_2-C_6H_4-4OCH_3$ |
| N | CH | N | H | $-CH_2-C_6H_3-3,4-(OCH_3)_2$ |
| N | CH | N | $CH_3$ | $-CH_2-C_6H_3-3,4-(OCH_3)_2$ |
| N | CH | N | H | $-CH_2-C_6H_4-4-OC_2H_5$ |
| N | CH | N | $CH_3$ | $-CH_2-C_6H_4-4-OC_2H_5$ |
| N | CH | N | H | $-CH_2-C_6H_4-4-OC_3H_7-n$ |
| N | CH | N | $CH_3$ | $-CH_2-C_6H_4-4-OC_3H_7-n$ |
| N | CH | N | H | $-CH_2-C_6H_4-4-OCH_2CH=CH_2$ |
| N | CH | N | $CH_3$ | $-CH_2-C_6H_4-4-OCH_2CH=CH_2$ |
| N | CH | N | H | $-CH_2-C_6H_4-4-NO_2$ |
| N | CH | N | H | $-CH_2-C_6H_4-4-CF_3$ |
| N | CH | N | $CH_3$ | $-CH_2-C_6H_4-4-CF_3$ |
| N | CH | N | H | $-CH_2-$(Thienyl-2) |
| N | CH | N | $CH_3$ | $-CH_2-$(Thienyl-2) |
| N | CH | N | H | $-CH_2-$(Thienyl-3) |
| N | CH | N | $CH_3$ | $-CH_2-$(Thienyl-3) |
| N | CH | N | H | $-CH_2-$(5-Chlor-thienyl-2) |
| N | CH | N | $CH_3$ | $-CH_2-$(5-Chlor-thienyl-2) |
| N | CH | N | $C_2H_5$ | $-CH_2-$(5-Chlor-thienyl-2) |
| N | CH | N | $C_4H_9-n$ | $-CH_2-$(5-Chlor-thienyl-2) |
| N | CH | N | H | $-CH_2-$(5-Chlor-thienyl-3) |
| N | CH | N | $CH_3$ | $-CH_2-$(5-Chlor-thienyl-3) |
| N | CH | N | $C_2H_5$ | $-CH_2-$(5-Chlor-thienyl-3) |
| N | CH | N | $C_3H_7-n$ | $-CH_2-$(5-Chlor-thienyl-3) |
| N | CH | N | H | $-CH_2-$(5-Brom-thienyl-2) |
| N | CH | N | H | $-CH_2-$(5-Brom-thienyl-3) |
| N | CH | N | H | $-CH_2-$(5-Methyl-thienyl-2) |
| N | CH | N | H | $-CH_2-$(5-Methyl-thienyl-3) |
| N | CH | N | H | $-CH_2-$(4,5-Dichlor-thienyl-2) |
| N | CH | N | $CH_3$ | $-CH_2-$(4,5-Dichlor-thienyl-2) |
| N | CH | N | H | $-CH_2-$(4,5-Dichlor-thienyl-3) |
| N | CH | N | $CH_3$ | $-CH_2-$(4,5-Dichlor-thienyl-3) |
| N | CH | N | H | $-CH_2-$(Furanyl-2) |
| N | CH | N | $CH_3$ | $-CH_2-$(Furanyl-2) |
| N | CH | N | H | $-CH_2-$(5-Methyl-furanyl-2) |
| N | CH | N | H | $-CH_2-$(Isoxazolyl-3) |

| X | Y | Z | $R^a$ | R |
|---|---|---|---|---|
| N | CH | N | H | $-CH_2-$(Isoxazolyl-4) |
| N | CH | N | H | $-CH_2-$(Isoxazolyl-5) |
| N | CH | N | H | $-CH_2-$(5-Methyl-isoxazolyl-3) |
| N | CH | N | H | $-CH_2-$(Pyridinyl-2) |
| N | CH | N | H | $-CH_2-$(Pyridinyl-3) |
| N | CH | N | H | $-CH_2-$(Pyridinyl-4) |
| N | CH | N | H | $-(CH_2)_2-C_6H_5$ |
| N | CH | N | H | $-CH_2-CH(CH_3)-C_6H_5$ |
| N | CH | N | H | $-CH_2-CH_2-O-C_6H_5$ |
| N | CH | N | H | $-CH_2-CH_2-S-C_6H_5$ |
| N | CH | N | H | $-(CH_2)_3C_6H_5$ |
| N | CH | N | $CH_3$ | $-(CH_3)_3C_6H_5$ |
| N | CH | N | H | $-CH_2-CH=CH-C_6H_5$ |
| N | CH | N | H | $-CH_2CH_2CH_2-O-C_6H_5$ |
| N | CH | N | $CH_3$ | $-CH_2CH_2CH_2-O-C_6H_5$ |
| N | CH | N | H | $-CH_2-CH(CH_3)-CH_2-C_6H_5$ |
| N | CH | N | H | $-CH_2-CH(CH_3)-CH_2-O-C_6H_5$ |
| N | CH | N | H | $-(CH_2)_4-O-C_6H_5$ |
| N | CH | N | H | $-(CH_2)_4-S-C_6H_5$ |
| N | CH | N | H | $-(CH_2)_4-C_6H_5$ |
| N | CH | N | $CH_3$ | $-(CH_2)_4-C_6H_5$ |
| N | CH | N | H | $-(CH_2)_4-C_6H_4-4F$ |
| N | CH | N | $CH_3$ | $-(CH_2)_4-C_6H_4-4F$ |
| N | CH | N | H | $-(CH_2)_4-C_6H_4-4CH_3$ |
| N | CH | N | H | $-(CH_2)_4-C_6H_4-4-OCH_3$ |
| N | CH | N | $CH_3$ | $-(CH_2)_4-C_6H_4-4-OCH_3$ |
| N | CH | N | H | $-(CH_2)_4-C_6H_4-4Cl$ |
| N | CH | N | H | $-(CH_2)_4-C_6H_4-4Br$ |
| N | CH | N | H | $-(CH_2)_4-C_6H_3-2,4-Cl_2$ |
| N | CH | N | $CH_3$ | $-(CH_2)_4-C_6H_3-2,4-Cl_2$ |
| N | CH | N | H | $-(CH_2)_4-C_6H_3-3,4-Cl_2$ |
| N | CH | N | $CH_3$ | $-(CH_2)_4-C_6H_3-3,4-Cl_2$ |
| N | CH | N | H | $-(CH_2)_4-C_6H_3-2,6-Cl_2$ |
| N | CH | N | H | $-(CH_2)_4-C_6H_3-2,4-(CH_3)_2$ |
| N | CH | N | H | $-(CH_2)_4-C_6H_3-3,4-(CH_3)_2$ |
| N | CH | N | H | $-(CH_2)_4-C_6H_3-2,6-(CH_3)_2$ |
| N | CH | N | H | $-(CH_2)_4-C_6H_4-4-NO_2$ |
| N | CH | N | H | $-CH_2-CH_2-CH=CH-C_6H_5$ |
| N | CH | N | $CH_3$ | $-CH_2-CH_2-CH=CH-C_6H_5$ |

| X | Y | Z | $R^a$ | R |
|---|---|---|---|---|
| N | CH | N | H | $-CH_2-CH_2-CH=CH-C_6H_4-4F$ |
| N | CH | N | $CH_3$ | $-CH_2-CH_2-CH=CH-C_6H_4-4F$ |
| N | CH | N | H | $-CH_2-CH_2-CH=CH-C_6H_4-4Cl$ |
| N | CH | N | $CH_3$ | $-CH_2-CH_2-CH=CH-C_6H_4-4Cl$ |
| N | CH | N | H | $-CH_2-CH_2-CH=CH-C_6H_4-4Br$ |
| N | CH | N | $CH_3$ | $-CH_2-CH_2-CH=CH-C_6H_4-4Br$ |
| N | CH | N | H | $-CH_2-CH=CH-CH_2-O-C_6H_5$ |
| N | CH | N | H | $-CH_2-C(CH_3)=CH-C_6H_5$ |
| N | CH | N | H | $-CH_2-C(C_2H_5)=CH-C_6H_5$ |
| N | CH | N | H | $-CH_2-CH=C(CH_3)-C_6H_5$ |
| N | CH | N | H | $-CH_2-CH=C(C_3H_7)-C_6H_5$ |
| N | CH | N | H | $-CH_2-CH=CH-C_6H_4-4F$ |
| N | CH | N | $CH_3$ | $-CH_2-CH=CH-C_6H_4-4F$ |
| N | CH | N | H | $-CH_2-CH=CH-C_6H_4-4Cl$ |
| N | CH | N | $CH_3$ | $-CH_2-CH=CH-C_6H_4-4Cl$ |
| N | CH | N | H | $-CH_2-CH=CH-C_6H_3-2,6-Cl_2$ |
| N | CH | N | $CH_3$ | $-CH_2-CH=CH-C_6H_3-2,6-Cl_2$ |
| N | CH | N | H | $-CH_2-CH=CH-C_6H_4-4-OCH_2$ |
| N | CH | N | H | $-CH_2-CH=CH-CH_2-$(Thienyl-2) |
| N | CH | N | H | $-(CH_2)_4$(Thienyl-2) |
| N | CH | N | H | $-(CH_2)_5-C_6H_5$ |
| N | CH | N | H | $-(CH_2)_6-C_6H_5$ |
| N | CH | N | H | $-(CH_2)_6-C_6H_4-4F$ |
| N | CH | N | H | $-(CH_2)_6-$(Pyridyl-3) |
| N | CH | N | H | $-(CH_2)_7-C_6H_5$ |
| N | CH | N | H | $-(CH_2)_8-C_6H_5$ |

Tabelle B

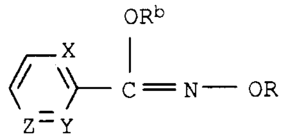

Ib

| X | Y | Z | $R^b$ | R |
|---|---|---|---|---|
| N | CH | N | $CH_3$ | $n-C_3H_7$ |
| N | CH | $N^+-O^-$ | $CH_3$ | $n-C_3H_7$ |
| N | CH | N | $C_2H_5$ | $n-C_3H_7$ |
| N | CH | N | $n-C_3H_7$ | $n-C_3H_7$ |
| N | CH | $N^+-O^-$ | $n-C_3H_7$ | $n-C_3H_7$ |
| N | CH | N | $n-C_4H_9$ | $n-C_3H_7$ |
| N | CH | N | $-CH_2CH=CH_2$ | $n-C_3H_7$ |
| CH | N | N | $CH_3$ | $n-C_3H_7$ |
| CH | N | $N^+-O^-$ | $CH_3$ | $n-C_3H_7$ |
| CH | N | N | $C_2H_5$ | $n-C_3H_7$ |
| CH | N | N | $n-C_3H_7$ | $n-C_3H_7$ |
| CH | N | N | $n-C_4H_9$ | $n-C_3H_7$ |
| CH | N | N | $-CH_2CH=CH_2$ | $n-C_3H_7$ |
| N | CH | N | $CH_3$ | $n-C_4H_9$ |
| N | CH | N | $C_2H_5$ | $n-C_4H_9$ |
| N | CH | N | $n-C_3H_7$ | $n-C_4H_9$ |
| N | CH | N | $CH_3$ | $iso-C_4H_9$ |
| N | CH | $N^+-O^-$ | $CH_3$ | $iso-C_4H_9$ |
| N | CH | N | $C_2H_5$ | $iso-C_4H_9$ |
| N | CH | N | $n-C_3H_7$ | $iso-C_4H_9$ |
| N | CH | N | $CH_3$ | $sek.-C_4H_9$ |
| CH | N | N | $CH_3$ | $sek.-C_4H_9$ |
| N | CH | N | $CH_3$ | $n-C_5H_{11}$ |
| N | CH | N | $CH_3$ | $iso-C_5H_{11}$ |
| N | CH | N | $CH_3$ | $n-C_6H_{13}$ |
| N | CH | N | $CH_3$ | $n-C_7H_{15}$ |
| CH | N | N | $CH_3$ | $n-C_7H_{15}$ |
| CH | N | N | $C_2H_5$ | $n-C_7H_{15}$ |
| N | CH | N | $CH_3$ | $n-C_8H_{17}$ |
| N | CH | $N^+-O^-$ | $CH_3$ | $n-C_8H_{17}$ |

| X | Y | Z | $R^b$ | R |
|---|---|---|---|---|
| N | CH | N | $CH_3$ | $-CH_2-CH_2-Cl$ |
| N | CH | N | $-CH_2CH=CH_2$ | $-CH_2-CH_2-Cl$ |
| N | CH | N | $CH_3$ | $-(CH_2)_3Cl$ |
| N | CH | N | $CH_3$ | $-(CH_2)_4Cl$ |
| N | CH | N | $CH_3$ | $-(CH_2)_5Cl$ |
| N | CH | N | $CH_3$ | $-(CH_2)_5Br$ |
| N | CH | N | $CH_3$ | $-(CH_2)_8Cl$ |
| CH | N | N | $CH_3$ | $-(CH_2)_8Cl$ |
| N | CH | N | $CH_3$ | $-CH_2-CF_3$ |
| N | N | CH | $CH_3$ | $-CH_2-CH_2OCH_3$ |
| CH | N | N | $CH_3$ | $-(CH_2)_3OCH_3$ |
| N | CH | N | $CH_3$ | $-(CH_2)_6OCH_3$ |
| N | CH | N | $CH_3$ | $-CH_2-CH=CH_2$ |
| N | CH | N | $C_2H_5$ | $-CH_2-CH=CH_2$ |
| N | CH | N | $CH_3$ | $-CH_2-C(CH_3)=CH_2$ |
| N | CH | N | $CH_3$ | $-CH_2-CH=C(CH_3)_2$ |
| N | CH | N | $CH_3$ | $-CH_2-C(Br)=CH_2$ |
| N | CH | N | $CH_3$ | $-CH_2-C(Cl)=CCl_2$ |
| CH | N | N | $CH_3$ | $-CH_2-C_6H_5$ |
| N | CH | N | $CH_3$ | $-CH_2-C_6H_5$ |
| N | CH | N | $C_2H_5$ | $-CH_2-C_6H_5$ |
| N | CH | N | $C_3H_7-n$ | $-CH_2-C_6H_5$ |
| N | CH | N | $CH_3$ | $-CH_2-C_6H_4-2F$ |
| N | CH | N | $C_2H_5$ | $-CH_2-C_6H_4-2F$ |
| N | CH | N | $CH_3$ | $-CH_2-C_6H_4-3F$ |
| N | CH | N | $CH_3$ | $-CH_2-C_6H_4-4F$ |
| N | CH | N | $C_4H_9-n$ | $-CH_2-C_6H_4-4F$ |
| N | CH | N | $CH_3$ | $-CH_2-C_6H_3-2,4-F_2$ |
| CH | N | N | $CH_3$ | $-CH_2-C_6H_4-2Cl$ |
| CH | N | N | $C_2H_5$ | $-CH_2-C_6H_4-2Cl$ |
| N | CH | N | $CH_3$ | $-CH_2-C_6H_4-2Cl$ |
| N | CH | N | $C_2H_5$ | $-CH_2-C_6H_4-2Cl$ |
| N | CH | N | $C_3H_7-n$ | $-CH_2-C_6H_4-2Cl$ |
| N | CH | N | $C_4H_9-n$ | $-CH_2-C_6H_4-2Cl$ |
| N | CH | N | $CH_3$ | $-CH_2-C_6H_4-3Cl$ |
| N | CH | N | $C_2H_5$ | $-CH_2-C_6H_4-3Cl$ |
| N | CH | N | $C_3H_7-n$ | $-CH_2-C_6H_4-3Cl$ |
| N | CH | N | $C_4H_9-n$ | $-CH_2-C_6H_4-3Cl$ |
| N | CH | N | $-CH_2CH=CH_2$ | $-CH_2-C_6H_4-3Cl$ |

| X | Y | Z | $R^b$ | R |
|---|---|---|---|---|
| N | CH | N | $CH_3$ | $-CH_2-C_6H_3-2,4-Cl_2$ |
| N | CH | N | $CH_3$ | $-CH_2-C_6H_3-3,4-Cl_2$ |
| CH | CH | N | $CH_3$ | $-CH_2-C_6H_3-2,6-Cl_2$ |
| N | CH | N | $CH_3$ | $-CH_2-C_6H_3-2,6-Cl_2$ |
| N | CH | N | $CH_3$ | $-CH_2-C_6H_4-4Br$ |
| N | CH | N | $CH_3$ | $-CH_2-C_6H_4-2CH_3$ |
| N | CH | N | $CH_3$ | $-CH_2-C_6H_4-3CH_3$ |
| N | CH | N | $CH_3$ | $-CH_2-C_6H_4-4CH_3$ |
| N | CH | N | $CH_3$ | $-CH_2-C_6H_4-4C_4H_9$-tert. |
| N | CH | N | $CH_3$ | $-CH_2-C_6H_4-2OCH_3$ |
| N | CH | N | $CH_3$ | $-CH_2-C_6H_4-4OCH_3$ |
| N | CH | N | $CH_3$ | $-CH_2-C_6H_4-4OCH_3$ |
| N | CH | N | $C_2H_5$ | $-CH_2-C_6H_4-4OCH_3$ |
| N | CH | N | $CH_3$ | $-CH_2-C_6H_3-3,4-(OCH_3)_2$ |
| N | CH | N | $CH_3$ | $-CH_2-C_6H_4-4-OC_2H_5$ |
| N | CH | N | $CH_3$ | $-CH_2-C_6H_4-4-OC_3H_7$-n |
| N | CH | N | $CH_3$ | $-CH_2-C_6H_4-4-OCH_2CH=CH_2$ |
| N | CH | N | $CH_3$ | $-CH_2-C_6H_4-4-CF_3$ |
| N | CH | N | $CH_3$ | $-CH_2-$(Thienyl-2) |
| N | CH | N | $CH_3$ | $-CH_2-$(Thienyl-3) |
| N | CH | N | $CH_3$ | $-CH_2-$(5-Chlor-thienyl-2) |
| N | CH | N | $C_2H_5$ | $-CH_2-$(5-Chlor-thienyl-2) |
| N | CH | N | $C_4H_9$-n | $-CH_2-$(5-Chlor-thienyl-2) |
| N | CH | N | $CH_3$ | $-CH_2-$(5-Chlor-thienyl-3) |
| N | CH | N | $C_2H_5$ | $-CH_2-$(5-Chlor-thienyl-3) |
| N | CH | N | $C_3H_7$-n | $-CH_2-$(5-Chlor-thienyl-3) |
| N | CH | N | $CH_3$ | $-CH_2-$(4,5-Dichlor-thienyl-2) |
| N | CH | N | $CH_3$ | $-CH_2-$(4,5-Dichlor-thienyl-3) |
| N | CH | N | $CH_3$ | $-CH_2-$(Furanyl-2) |
| N | CH | N | $CH_3$ | $-(CH_3)_3C_6H_5$ |
| N | CH | N | $CH_3$ | $-CH_2CH_2CH_2-O-C_6H_5$ |
| N | CH | N | $CH_3$ | $-(CH_2)_4-C_6H_5$ |
| N | CH | N | $CH_3$ | $-(CH_2)_4-C_6H_4-4F$ |
| N | CH | N | $CH_3$ | $-(CH_2)_4-C_6H_4-4-OCH_3$ |
| N | CH | N | $CH_3$ | $-CH_2-CH_2-CH=CH-C_6H_5$ |
| N | CH | N | $CH_3$ | $-CH_2-CH_2-CH=CH-C_6H_4-4F$ |
| N | CH | N | $CH_3$ | $-CH_2-CH_2-CH=CH-C_6H_4-4Cl$ |
| N | CH | N | $CH_3$ | $-CH_2-CH_2-CH=CH-C_6H_4-4Br$ |
| N | CH | N | $CH_3$ | $-CH_2-CH=CH-C_6H_4-4F$ |

| X | Y | Z | $R^b$ | R |
|---|---|---|---|---|
| N | CH | N | $CH_3$ | $-CH_2-CH=CH-C_6H_4-4Cl$ |
| N | CH | N | $CH_3$ | $-CH_2-CH=CH-C_6H_3-2,6-Cl_2$ |

Die Verbindungen Ia und Ib zeichnen sich durch eine hervorragende Wirkung gegen ein breites Spektrum von pflanzenpathogenen Pilzen, insbesondere aus der Klasse der Ascomyceten und Basidiomyceten, aus. Sie sind zT. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Baumwolle, Gemüsepflanzen (zB. Gurken, Bohnen und Kürbisgewächse), Gerste, Gras, Hafer, Kaffee, Mais, Obstpflanzen, Reis, Roggen, Soja, Wein, Weizen, Zierpflanzen, Zuckerrohr und einer Vielzahl von Samen.

Insbesondere eignen sie sich zur Bekämpfung der folgenden pflanzenpathogenen Pilze: Erysiphe graminis (echter Mehltau) an Getreide, Erysiphe cichoracearum und Sphaerotheca fuliginea an Kürbisgewächsen, Podosphaera leucotricha an Äpfeln, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an getreide, Septoria nodorum an Weizen, Botrytis cinerea (Grauschimmel) an Erdbeeren und Reben, Cercospora arachidicola an Erdnüssen, Pseudocercosporella herpotrichoides an Weizen und Gerste, Pyricularia oryzae an Reis, Phytophthora infestans an Kartoffeln und Tomaten, Plasmopara viticola an Reben, Alternaria-Arten an Gemüse und Obst sowie Fusarium- und Verticillium-Arten.

Sie sind außerdem im Materialschutz (z.B. Holzschutz) anwendbar, beispielsweise gegen Paecilomyces variotii.

Die Aufwandmengen der erfindungsgemäßen Verbindungen liegen je nach Art des gewünschten Effekts bei 0,02 bis 3 kg/ha, vorzugsweise 0,1 bis 2 kg/ha, insbesondere 0,5 bis 1 kg/ha.

Bei der Saatgutbehandlung werden im allgemeinen Aufwandmengen an den Verbindungen Ia bzw. Ib von 0,001 bis 50 g/kg Saatgut, vorzugsweise 0,01 bis 10 g/kg, insbesondere 0,01 bis 5 g/kg, verwendet.

Sofern für Pflanzen pathogene Schadpilze zu bekämpfen sind erfolgt die Applikation der Verbindungen Ia bzw. Ib durch Besprühen oder Bestäuben der Samen, der Pflanzen oder der Böden vor oder nach der Aussaat der Pflanzen oder vor oder nach dem Auflaufen der Pflanzen.

Die erfindungsgemäßen fungiziden Mittel bzw. die Verbindungen Ia und Ib können beispielsweise in Form von direkt versprügbaren Lösungen, Pulver und Suspensionen oder in Form von hochprozentigen wäßrigen, öligen oder sonstigen Suspensionen, Dispersionen, Emulsionen, Öldispersionen, Pasten, Stäubemitteln, Streumitteln oder Granulaten aufbereitet und durch Versprühen, Vernebeln, Verstäuben, Verstreuen oder Gießen angewendet werden. Die Anwendungsform ist abhängig vom Verwendungszweck; sie soll in jedem Fall eine möglichst feine und gleichmäßige Verteilung der erfindungsgemäßen Mischung gewährleisten.

Die Formulierungen werden in an sich bekannter Weise hergestellt, zB. durch Zugabe von Lösungsmitteln und/oder Trägerstoffen. Den Formulierungen werden üblicherweise inerte Zusatzstoffe wie Emulgiermittel oder Dispergiermittel beigemischt.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole oder Fettalkoholglycolethern, Kondensationsprodukte von sulfoniertem Naphthalin und seinen Derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol- oder Tributylphenylpolyglycolether, Alkylarylpolyetheralkohole, Isotridecylalkohol, Fettalkoholethylenoxid- Kondensate, ethoxyliertes Rizinusöl, Polyoxyethylenalkylether oder Polyoxypropylenalkylether , Laurylalkoholpolyglycoletheracetat, Sorbitester, Lignin-Sulfitablaugen oder Methylcellulose in Betracht.

Pulver, Streu- und Stäubemittel können durch Mischen oder gemeinsames Vermahlen der Verbindungen Ia oder Ib oder der Mischung aus den Verbindungen Ia und Ib mit einem festen Trägerstoff hergestellt werden.

Granulate (zB. Umhüllungs-, Imprägnierungs- oder Homogengranulate) werden üblicherweise durch Bindung des Wirkstoffs oder der Wirkstoffe an einen festen Trägerstoff hergestellt.

Als Füllstoffe bzw. feste Trägerstoffe dienen beispielsweise Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Kalzium- und Magnesiumsulfat, Magnesiumoxid, gemahlene Kunststoffe, sowie Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen 0,1 bis 95 Gew.-%, vorzugsweise 0,5 bis 90 Gew.-% der Verbindungen Ia oder Ib. Die Wirkstoffe werden dabei in einer Reinheit von 90 % bis 100 %, vorzugsweise 95 % bis 100 % (nach NMR oder HPLC-Spektrum) eingesetzt.

Beispiele für solche Zubereitungen sind:

I. Man vermischt 90 Gew.-Teile der Verbindung gemäß Beispiel 1.01 mit 10Gew.-Teilen N-Methyl-α-pyrrolidon und erhält eine Lösung, die zur Anwendung in Form kleinster Tropfen geeignet ist.

II. 20 Gew.-Teile der Verbindung gemäß Beispiel 1.19 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1.25 aus Tabelle 1A werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1.26 aus Tabelle 1A werden in einer Mischung gelöst, die aus 25 Gew.-Teilen Cyclohexanol, 65 Gew.-Teilen einer Mineralölfraktion vom Siedepunkt 210 bis 280 °C und 10Gew.-Teilen des Anlagerungsproduktes von 40 Mol Ethylenoxid an 1Mol Ricinusöl besteht. Durch Eingießen und feines Verteilen der Lösung in Wasser erhält man eine wäßrige Dispersion.

V. 80 Gew.-Teile der Verbindung Nr. 1.56 aus Tabelle 1A werden mit 3Gew.-Teilen des Natriumsalzes der Diisobutylnaphthalin-α-sulfon säure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1.57 aus Tabelle 1A werden mit 97Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung Nr. 1.60 aus Tabelle 1A werden mit einer Mischung aus 92 Gew.-Teilen pulverförmigem Kieselsäuregel und 8Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2.20 aus Tabelle 1A werden mit 10Gew.-Teilen Natriumsalz eines Phenolsulfonsäureharnstoffformaldehyd-Kondensats, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2.11 aus Tabelle 1B werden mit 2Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die Verbindungen Ia und Ib bzw. die entsprechenden Formulierungen werden angewendet, indem man die Schadpilze, die von ihnen freizuhaltenden Pflanzen, Samen, Böden, Flächen, Materialien oder Räume mit einer fungizid wirksamen Menge der Verbindungen Ia bzw. Ib behandelt. Die Anwendung kann vor oder nach dem Befall durch die Schadpilze erfolgen.

Neben der Verwendung von Formulierungen der reinen Wirkstoffe ist es auch möglich, die Wirkstoffe bereits bei der Formulierung oder unmittelbar vor der Applikation (Tankmix) mit anderen Wirkstoffen zu mischen. Als Mischungspartner kommen andere fungizide Wirkstoffe oder auch Herbizide, Wirkstoffe zu Schädlingsbekämpfung (Insektizide, Nematizide, Akarizide), Wachstumsregulatoren oder Düngemittel in Betracht. Diese Beimischung von anderen Wirkstoffen, insbesondere anderen fungiziden Wirkstoffen, kann eine zusätzliche Wirkungssteigerung bzw. eine Verbreiterung des Wirkungsspektrums zur Folge haben. Üblicherweise werden die weiteren Wirkstoffe in einem Verhältnis von 10:1 bis 0,1:1 Gew.-%, vorzugsweise 5:1 bis 0,2:1 Gew.-%, bezogen auf die Verbindungen Ia bzw. Ib zugesetzt.

Synthesebeispiele

Die in den nachstehenden Synthesebeispielen wiedergegebenen Vorschriften zur Herstellung der Verbindungen Ia bzw. Ib können unter Abwandlung der Ausgangsverbindungen zur Gewinnung weiterer Vertreter der Verbindungen Ia und Ib benutzt werden. Demgemäß hergestellte Beispiele sind in den anschließenden Tabellen mit physikalischen Daten angegeben.

Beispiel 1

N-(Benzyloxy)-pyrazincarbonsäureamid [Nr. 1.01]

Eine Mischung aus 12,4 g (0,089 mol) Pyrazin-carbonsäure-methylester, 22,3 g (0,180 mol) O-Benzylhydroxylamin und 5 g 4-Dimethylaminopyridin wurde 20 h bei 70 °C gerührt. Nach dem Abkühlen der Reaktionsmischung auf 30 °C wurde mit 50 ml Wasser versetzt und die organische Phase wurde abgetrennt. Die isolierte organische Phase wurde mit 30 ml Diethylether verrührt. Das bei 0 °C auskristallisierende Produkt wurde isoliert, mit Diethylether gewaschen und getrocknet. Man erhielt 10,4 g der Verbindung Nr. 1.01 als weiße Kristalle; Schmp. 90-92 °C.

Beispiel 2

N-(Benzyloxy)-N-methyl-pyrazincarbonsäureamid [Nr. 1.02] und N-(Benzyloxy)-iminopyrazincarbonsäure-O-methylester [Nr. 2.01]

Eine Lösung von 23,6 g (0,103 mol) N-(Benzyloxy)-pyrazincarbonsäureamid in 150 ml Aceton wurde nacheinander mit 27,6 g (0,2 mol) Kaliumcarbonat und mit 13,8 g (0,11 mol) Dimethylsulfat versetzt. Nach 12 h Rühren bei 25 °C und anschließendem vierstündigem Rühren bei 55 °C ließ man die Reaktionsmischung auf 20 °C abkühlen. Der anorganische Feststoff wurde abfiltriert. Aus der eingeengten Reaktionslösung erhielt man nach "Flash-Chromatographie" [Kieselgel "60" (Korngröße ≦ 0,063 mm); Methylenchlorid]

a) 2,4 g der Verbindung Nr. 1.02 als farbloses Öl; $n^{23}_D$ = 1,5702 und

b) 4,7 g der Verbindung Nr. 2.01 als farbloses Öl; $n^{23}_D$ = 1,5830.

Tabelle 1A

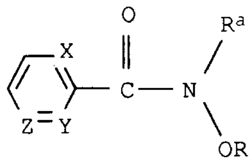

Ia

| Nr. | X | Y | Z | $R^a$ | R | phys.Daten [Fp (°C); $n_D^{23}$] |
|---|---|---|---|---|---|---|
| 1.01 | N | CH | N | H | $CH_2-C_6H_5$ | 90-92 |
| 1.02 | N | CH | N | $CH_3$ | $CH_2-C_6H_5$ | 1,5702 |
| 1.03 | N | CH | N | H | $(CH_2)_2CH_3$ | 90-91 |
| 1.04 | N | CH | N | H | $CH(CH_3)_2$ | 71-73 |
| 1.05 | N | CH | N | H | $CH_2CH(CH_3)_2$ | 75-77 |
| 1.06 | N | CH | N | H | $CH_2C(CH_3)=CH_2$ | 67-69 |
| 1.07 | N | CH | N | H | $CH(CH_3)-C_6H_5$ | 110-111 |
| 1.08 | N | CH | N | H | $CH_2CH=CHCH_2-C_6H_5$ | 81-83 |
| 1.09 | N | CH | N | $CH_3$ | $CH_2CH=CHCH_2-C_6H_5$ | 1,5678 |
| 1.10 | N | CH | N | H | $CH_2-(2-F-C_6H_4)$ | 106-107 |
| 1.11 | N | CH | N | H | $(CH_2)_2O-(2-F-C_6H_4)$ | 109-111 |
| 1.12 | N | CH | N | $CH_3$ | $(CH_2)_2O-(2-F-C_6H_4)$ | 1,5546 |
| 1.13 | N | CH | N | H | $CH_2-(3-F-C_6H_4)$ | 111-112 |
| 1.14 | N | CH | N | $CH_3$ | $CH_2-(3-F-C_6H_4)$ | 66-67 |
| 1.15 | N | CH | N | H | $(CH_2)_4-(4-F-C_6H_4)$ | 51-53 |
| 1.16 | N | CH | N | $CH_3$ | $(CH_2)_4-(4-F-C_6H_4)$ | 1,5397 |
| 1.17 | N | CH | N | H | $(CH_2)_2CH=CH-(4-F-C_6H_4)$ | 75-76 |
| 1.18 | N | CH | N | $CH_3$ | $(CH_2)_2CH=CH-(4-F-C_6H_4)$ | 1,5678 |
| 1.19 | N | CH | N | H | $CH_2-(2-Cl-C_6H_4)$ | 100-102 |
| 1.20 | N | CH | N | $CH_3$ | $CH_2-(2-Cl-C_6H_4)$ | 90-91 |
| 1.21 | N | CH | N | H | $CH_2-(3-Cl-C_6H_4)$ | 84-87 |
| 1.22 | N | CH | N | $CH_3$ | $CH_2-(3-Cl-C_6H_4)$ | 1,5778 |
| 1.23 | N | CH | N | H | $(CH_2)_2O-(3-Cl-C_6H_4)$ | 103-105 |
| 1.24 | N | CH | N | $CH_3$ | $(CH_2)_2O-(3-Cl-C_6H_4)$ | 1,5706 |
| 1.25 | N | CH | N | H | $CH_2-(4-Cl-C_6H_4)$ | 130-132 |
| 1.26 | N | CH | N | $CH_3$ | $CH_2-(4-Cl-C_6H_4)$ | 49-50 |
| 1.27 | N | CH | N | H | $CH(CH_3)-(4-Cl-C_6H_4)$ | 134-135 |
| 1.28 | N | CH | N | H | $(CH_2)_4-(4-Cl-C_6H_4)$ | 105-106 |
| 1.29 | N | CH | N | H | $(CH_2)_2CH=CH-(4-Cl-C_6H_4)$ | 110-111 |

| Nr. | X | Y | Z | $R^a$ | R | phys.Daten [Fp (°C); $n_D^{23}$] |
|---|---|---|---|---|---|---|
| 1.30 | N | CH | N | $CH_3$ | $(CH_2)_2CH{=}CH-(4-Cl-C_6H_4)$ | 83-84 |
| 1.31 | N | CH | N | H | $CH_2-CH{=}CH-CH_2-(4-Cl-C_6H_4)$ | 80-82 |
| 1.32 | N | CH | N | $CH_3$ | $CH_2-CH{=}CH-CH_2-(4-Cl-C_6H_4)$ | 1,5729 |
| 1.33 | N | CH | N | H | $CH_2-(3,4-Cl_2-C_6H_3)$ | 130-131 |
| 1.34 | N | CH | N | $CH_3$ | $CH_2-(3,4-Cl_2-C_6H_3)$ | 67-68 |
| 1.35 | N | CH | N | H | $(CH_2)_4-(2,6-Cl_2-C_6H_3)$ | 83-84 |
| 1.36 | N | CH | N | H | $CH_2CH{=}CHCH_2-(2,6-Cl_2-C_6H_3)$ | 70-71 |
| 1.37 | N | CH | N | $CH_3$ | $CH_2CH{=}CHCH_2-(2,6-Cl_2-C_6H_3)$ | 70-71 |
| 1.38 | N | CH | N | H | $CH_2-(3-Br-C_6H_4)$ | 90-91 |
| 1.39 | N | CH | N | $CH_3$ | $CH_2-(3-Br-C_6H_4)$ | 1,5912 |
| 1.40 | N | CH | N | H | $(CH_2)_2CH{=}CH-(4-Br-C_6H_4)$ | 114-115 |
| 1.41 | N | CH | N | $CH_3$ | $(CH_2)_2CH{=}CH-(4-Br-C_6H_4)$ | 67-68 |
| 1.42 | N | CH | N | H | $CH_2-(2-CH_3-C_6H_4)$ | 101-102 |
| 1.43 | N | CH | N | H | $CH_2-(2-CH_3-C_6H_4)$ | 1,5722 |
| 1.44 | N | CH | N | H | $CH_2-(3-CH_3-C_6H_4)$ | 220-222 |
| 1.45 | N | CH | N | $CH_3$ | $CH_2-(3-CH_3-C_6H_4)$ | 1,5681 |
| 1.46 | N | CH | N | H | $CH_2-(4-CH_3-C_6H_4)$ | 137-140 |
| 1.47 | N | CH | N | $CH_3$ | $CH_2-(4-CH_3-C_6H_4)$ | 1,5655 |
| 1.48 | N | CH | N | H | $CH_2-(4-OCH_3-C_6H_4)$ | 131-132 |
| 1.49 | N | CH | N | $CH_3$ | $CH_2-(4-OCH_3-C_6H_4)$ | 1,5652 |
| 1.50 | N | CH | N | H | $(CH_2)_4-(4-OCH_3-C_6H_4)$ | 58- 60 |
| 1.51 | N | CH | N | $CH_3$ | $(CH_2)_4-(4-OCH_3-C_6H_4)$ | 1,5533 |
| 1.52 | N | CH | N | H | $(CH_2)_4-(3-OCH_3-C_6H_4)$ | 68- 70 |
| 1.53 | N | CH | N | $CH_3$ | $(CH_2)_4-(3-OCH_3-C_6H_4)$ | 1,5520 |
| 1.54 | N | CH | N | H | $CH_2$-(thien-3-yl) | 125-126 |
| 1.55 | N | CH | N | $CH_3$ | $CH_2$-(thien-3-yl) | 1,5859 |
| 1.56 | N | CH | N | H | $CH_2$-(5-Cl-thien-3-yl) | 103-104 |
| 1.57 | N | CH | N | $CH_3$ | $CH_2$-(5-Cl-thien-3-yl) | 1,5887 |
| 1.58 | N | CH | N | H | $(CH_2)_4$-(5-$CH_3$-thien-2-yl) | 73- 75 |
| 1.59 | N | CH | N | $CH_3$ | $(CH_2)_4$-(5-$CH_3$-thien-2-yl) | 1,5543 |
| 1.60 | N | CH | N | H | $CH_2$-(4,5-$Cl_2$-thien-3-yl) | 115-116 |
| 1.61 | N | CH | N | $CH_3$ | $CH_2$-(4,5-$Cl_2$-thien-3-yl) | 106-107 |

Tabelle 1B

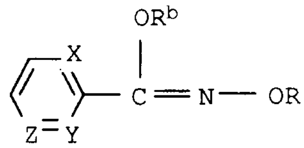

Ib

| Nr. | X | Y | Z | $R^b$ | R | phys.Daten [Fp (°C); $n_D^{23}$] |
|---|---|---|---|---|---|---|
| 2.01 | N | CH | N | $CH_3$ | $CH_2$-$C_6H_5$ | 1,5830 |
| 2.02 | N | CH | N | $CH_3$ | $CH_2CH$=$CHCH_2$-$C_6H_5$ | 1,5745 |
| 2.03 | N | CH | N | $CH_3$ | $(CH_2)_2O$-(2-F-$C_6H_4$) | 1,5608 |
| 2.04 | N | CH | N | $CH_3$ | $CH_2$-(3-F-$C_6H_4$) | 1,5662 |
| 2.05 | N | CH | N | $CH_3$ | $(CH_2)_4$-(4-F-$C_6H_4$) | 1,5474 |
| 2.06 | N | CH | N | $CH_3$ | $(CH_2)_2CH$=CH-(4-F-$C_6H_4$) | 1,5772 |
| 2.07 | N | CH | N | $CH_3$ | $CH_2$-(2-Cl-$C_6H_4$) | 1,5900 |
| 2.08 | N | CH | N | $CH_3$ | $CH_2$-(3-Cl-$C_6H_4$) | 1,5878 |
| 2.09 | N | CH | N | $CH_3$ | $(CH_2)_2O$-(3-Cl-$C_6H_4$) | 69- 71 |
| 2.10 | N | CH | N | $CH_3$ | $CH_2$-(4-Cl-$C_6H_4$) | 73- 76 |
| 2.11 | N | CH | N | $CH_3$ | $(CH_2)_2CH$=CH-(4-Cl-$C_6H_4$) | 1,5998 |
| 2.12 | N | CH | N | $CH_3$ | $CH_2CH$=$CHCH_2$-(4-Cl-$C_6H_4$) | 1,5820 |
| 2.13 | N | CH | N | $CH_3$ | $CH_2$-(3,4-$Cl_2$-$C_6H_3$) | 91- 92 |
| 2.14 | N | CH | N | $CH_3$ | $CH_2CH$=$CHCH_2$-(2,6-$Cl_2$-$C_6H_3$) | 82- 83 |
| 2.15 | N | CH | N | $CH_3$ | $CH_2$-(3-Br-$C_6H_4$) | 1,6000 |
| 2.16 | N | CH | N | $CH_3$ | $(CH_2)_2CH$=CH-(4-Br-$C_6H_4$) | 1,6128 |
| 2.17 | N | CH | N | $CH_3$ | $CH_2$-(2-$CH_3$-$C_6H_4$) | 1,5800 |
| 2.18 | N | CH | N | $CH_3$ | $CH_2$-(3-$CH_3$-$C_6H_4$) | 1,5732 |
| 2.19 | N | CH | N | $CH_3$ | $CH_2$-(4-$CH_3$-$C_6H_4$) | 1,5748 |
| 2.20 | N | CH | N | $CH_3$ | $CH_2$-(4-$OCH_3$-$C_6H_4$) | 1,5785 |
| 2.21 | N | CH | N | $CH_3$ | $(CH_2)_4$-(4-$OCH_3$-$C_6H_4$) | 1,5612 |
| 2.22 | N | CH | N | $CH_3$ | $(CH_2)_4$-(3-$OCH_3$-$C_6H_4$) | 1,5602 |
| 2.23 | N | CH | N | $CH_3$ | $CH_2$-(thien-3-yl) | 1,5962 |
| 2.24 | N | CH | N | $CH_3$ | $CH_2$-(5-Cl-thien-3-yl) | 1,5989 |
| 2.25 | N | CH | N | $CH_3$ | $(CH_2)_4$-(5-$CH_3$-thien-2-yl) | 1,5568 |
| 2.26 | N | CH | N | $CH_3$ | $CH_2$-(4,5-$Cl_2$-thien-3-yl) | 1,6043 |

Anwendungsbeispiele:

Die fungizide Wirkung der Verbindungen der allgemeinen Formel Ia und Ib ließ sich durch folgende Versuche zeigen:

Anwendungsbeispiel 1

Wirksamkeit gegen Botrytis cinerea

Paprikasämlinge der Sorte "Neusiedler Ideal Elite" wurden, nachdem sich 4-5 Blätter gut entwickelt hatten, mit wäßrigen Suspension, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

In diesem Test zeigten die mit 500 ppm der Verbindungen 1.01, 1.08, 1.13, 1.19, 1.22, 1.25, 1.43, 1.56 und 1.58 behandelten Pflanzen einen Befall von 15 % und weniger, während bei den unbehandelten Pflanzen 90 % der Blattfläche befallen waren.

Anwendungsbeispiel 2

Wirksamkeit gegen Phytophthora infestans an Tomaten

Blätter von Tomatenpflanzen der Sorte "Große Fleischtomate" wurden mit wäßriger Spritzbrühe, die 80 % Wirkstoff und 20 % Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer wasserdampfgesättigen Kammer bei Temperaturen zwischen 16 und 18 °C aufgestellt. Nach 6 Tagen hat sich die Krankheit auf den unbehandelten, jedoch infizierten Kontrollpflanzen so stark entwickelt, daß die fungizide Wirksamkeit der Substanzen beurteilt werden konnte.

In diesem Text zeigten die mit 250 ppm der Verbindungen 1.30, 2.06 und 2.21 behandelten Pflanzen einen Befall von 15 %, während bei den unbehandelten Pflanzen 80 % der Blattfläche befallen waren.

## Patentansprüche

**1.** Pyridin- und Diazincarbonsäurederivate der Formeln Ia und Ib,

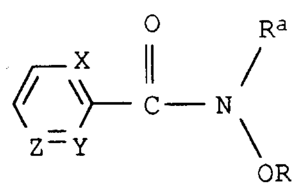

Ia

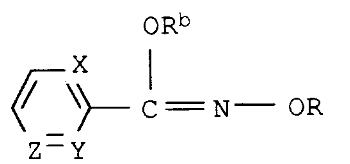

Ib

in der die Substituenten die folgende Bedeutung haben:

| | |
|---|---|
| X, Y und Z | CH oder N, wobei eine oder zwei der Variablen CH bedeutet und eine der Variablen zusätzlich $N^+$-$O^-$ bedeuten kann und wobei Z nicht N oder $N^+$-$O^-$ bedeutet, wenn X und Y gleichzeitig für CH stehen; |
| R | $C_3$-$C_8$-Alkyl, $C_2$-$C_8$-Halogenalkyl, $C_3$-$C_8$-Alkenyl, $C_3$-$C_8$-Halogenalkenyl oder $C_3$-$C_8$-Alkinyl; $C_1$-$C_{10}$-Alkyl oder $C_3$-$C_{10}$-Alkenyl, welches einen Aryl-, Aryloxy-, Arylthio-, Heteroaryl-, Heteroaryloxy- oder Heteroarylthiorest trägt, wobei der Aryl- bzw. der Heteroarylrest seinerseits ein bis fünf Halogenatome und/oder ein bis drei der folgenden Gruppen tragen kann: Nitro, Cyano, $C_1$-$C_4$-Alkyl, $C_1$-$C_4$-Halogenalkyl, $C_3$-$C_4$-Alkenyl, $C_1$-$C_4$-Alkoxy, $C_1$-$C_4$-Halogenalkoxy, $C_3$-$C_4$-Alkenyloxy oder $C_1$-$C_4$-Alkylthio, |
| $R^a$ | Wasserstoff, $C_1$-$C_4$-Alkyl, $C_3$-$C_4$-Alkenyl oder $C_3$-$C_4$-Alkinyl; |
| $R^b$ | $C_1$-$C_4$-Alkyl, $C_3$-$C_4$-Alkenyl oder $C_3$-$C_4$-Alkinyl, |

sowie deren Salze.

**2.** Verbindungen der Formeln Ia und Ib gemäß Anspruch 1, in denen R für $C_3$-$C_8$-Alkyl, $C_2$-$C_8$-Halogenalkyl, $C_3$-$C_8$-Alkenyl, $C_3$-$C_8$-Halogenalkenyl, $C_3$-$C_8$-Alkinyl oder $C_3$-$C_8$-Halogenalkinyl, oder für $C_1$-$C_{10}$-Alkyl oder $C_3$-$C_{10}$-Alkenyl steht, welches einen Phenyl-, Phenoxy-, Thienyl-, Thienyloxy-,

Furanyl-, Furanyloxy-, Isoxazolyl-, 2-Pyridyl- oder 2-Pyridyloxyrest trägt, wobei dieser Rest seinerseits ein bis fünf Halogenatome und/oder ein bis drei der folgenden Gruppen tragen kann: Nitro, Cyano, $C_1$-$C_4$-Alkyl, $C_1$-$C_4$-Halogenalkyl, $C_3$-$C_4$-Alkenyl, $C_1$-$C_4$-Alkoxy, $C_1$-$C_4$-Halogenalkoxy, $C_3$-$C_4$-Alkenyloxy oder $C_1$-$C_4$-Alkylthio.

**3.** Verbindungen der Formeln Ia und Ib gemäß Anspruch 1, in denen der X, Y und Z enthaltende Ring für Pyridin-2-yl, Pyridazin-3-yl, Pyrazinyl, Pyrimidin-2-yl, 1,4-Diazin-2-yl, Pyridin-2-yl- 1-N-oxid, Pyridazin-3-yl-1-N-oxid, Pyrazin-3-yl-1-N-oxid oder 1,4-Diazin-2-yl-4-N-oxid steht.

**4.** Verfahren zur Herstellung der Verbindungen der Formel Ia gemäß Anspruch 1, dadurch gekennzeichnet, daß man ein Hydroxylamin der Formel II

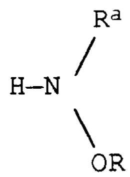

II

in an sich bekannter Weise mit einem Carbonsäurederivat der Formel III

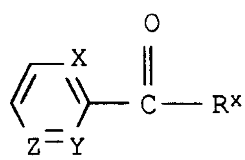

III

in der $R^x$ für ein Halogenatom oder eine $C_1$-$C_2$-Alkoxygruppe steht, umsetzt.

**5.** Verfahren zur Herstellung der Verbindungen der Formel Ib gemäß Anspruch 1, dadurch gekennzeichnet, daß man eine Verbindung der Formel Ia gemäß Anspruch 1 in an sich bekannter Weise mit einer Verbindung der Formel IV

$R^b$-$R^y$ IV

in der $R^y$ für eine nukleophil verdrängbare Abgangsgruppe steht, umsetzt.

**6.** Mittel gegen Schadpilze, enthaltend übliche Zusatzstoffe und eine wirksame Menge einer Verbindung der Formel Ia oder Ib gemäß Anspruch 1.

**7.** Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer wirksamen Menge einer Verbindung der Formel Ia oder Ib gemäß Anspruch 1 behandelt.

**8.** Verwendung der Verbindungen Ia bzw. Ib gemäß Anspruch 1 zur Herstellung von Mitteln gegen Schadpilze.

**9.** Verwendung der Verbindungen Ia bzw. Ib gemäß Anspruch 1 zur Bekämpfung von Schadpilzen.